# EUROPEAN PATENT APPLICATION

(11) **EP 4 385 451 A1**
(43) Date of publication of application: **19.06.2024**
(21) Application number: 23214027.7
(22) Date of filing: 04.12.2023
(51) Int. Cl.: A61B 90/14

(54) **POSITIONING SYSTEM OF A STEREOTACTIC FRAME**

(30) Priority: 07.12.2022 LT 2022549
(71) Applicant: Kauno Technologijos Universitetas, 44029 Kaunas (LT); Lietuvos sveikatos mokslu universitetas, 44307 Kaunas (LT)
(72) Inventor: Ostasevicius, Vytautas, Kaunas (LT); Bubulis, Algimantas, Kaunas (LT); Jurenas, Vytautas, Kaunas (LT); Eidukynas, Darius, Kaunas (LT); Radziunas, Andrius, Kaunas (LT); Tamasauskas, Arimantas, Kaunas (LT); Tamasauskas, Sarunas, Kaunas (LT)
(74) Representative: AAA Law

(57) **Abstract**

The invention belongs to medical field and is related to a locking device for immobilizing a patient's head, to treatment equipment or a medical device for performing treatment procedures in the head area. The stereotactic frame positioning system, which comprises soft inflatable silicone contact cushions for attaching the stereoscopic frame to the human head, are attached to inside side of the front side and inside of the back side of the stereoscopic frame and are connected via flexible tubes (hoses) to hand-operated air supply pumps. The air supply to the cushions can be adjusted through supply-discharge valves. Locking position of the frame in relation to the head of the human (patient) is done manually by applying compressed air to soft contact inflatable cushions, depending on the nature of the treatment or operation.

## Description

### TECHNICAL FIELD

The invention belongs to the medical field and relates to fixation devices for immobilizing the patient's head during treatment procedures in the head area.

### BACKGROUND ART

Stereotactic techniques are routinely used in neurosurgery for operations, such as aspiration of brain cysts, tumor biopsies, and, more recently, implantation of electrodes into brain targets.

An integral part of this operation is a mechanical device called a stereotactic frame, which contains head-holding clamps and rods that immobilize the head in a fixed position relative to a coordinate system.

There is a known US patent No.: US 8,100,132B2, which describes a locking device for immobilizing patient's head during treatment procedures in the head region with a medical device. The locking device comprises an interface hardware unit adapted to be attached to an adapter for connection to a treatment device or to a frame for connection to a medical device. In addition, the locking device has a sliding plate which slides in the XZ plane of the XYZ coordinate system with respect to said interface equipment. The locking device further comprises plate locking means for locking the sliding plate relative to said interface equipment unit, a biting device for insertion into the patient's mouth, coupling means for connecting said biting device to said sliding plate, at least one pivot assembly arranged so that the biting device can move in a plane perpendicular to the XZ plane; and coupling locking means for locking at least one pivot assembly in a desired position relative to said coupling means to lock the biting device to the interface equipment. In the specified device, the locking function is performed with the help of a biting device, which is designed to insert the sliding plate into the patient's mouth. Such immobilization of the locking device in relation to the human head depends on the patient's bite coordination abilities, i.e. not all patients are able to stably maintain a movable plate in the mouth during the bite, which complicates the success of treatment or operation.

There is known US patent No. US 8,944,064B2, which discloses a stereotactic frame comprising two or more arms, each of which is connected to the other by a point connection assembly at their point of intersection, and at least one of which has a plurality of adjustable means adapted to position and fix the position of the human head relative to the stereotactic frame. The fixing and adjustment in relation to the human head of specified structure of the stereotactic frame, i.e. arm system, is quite complicated and requires a lot of time to prepare for the planned operations.

There is a known surgical head frame disclosed in US patent application no. US 2002/0042619 A1 for supporting a reference frame of a stereotactic system relative to a patient's head, a human head locking frame consisting of a frame body and two brackets attached from opposite sides of the frame body, and contact cushions connected to the frame body and a strap configured to be placed on the patient's head and fixing the reference frame of the stereotactic system in relation to the patient's head. The large number of nodes and elements that make up this device of stereotactic system and the complex procedure of their adjustment reduce the success of the treatment and operations performed on patients.

The above-mentioned devices of the stereotactic system designed to maintain a stable position of the human head during treatment or operation have one common drawback, i.e. the design of the head frame and its adjustment system are too complicated.

### SUMMARY OF THE INVENTION

The invention is a stereotactic frame positioning system that includes a human head locking frame and soft inflatable cushions attached to inside of the frame, which are connected to upper air supply pumps through flexible tubes (hoses). Air supply to the cushions can be adjusted through valves. Locking position of the frame in relation to the head is done manually by applying compressed air to soft inflatable cushions, depending on the nature of the treatment or operation.

### SHORT DESCRIPTION OF DRAWINGS

The invention is explained in the drawings. The accompanying diagrams and drawings make an integral part of the invention description and are given as a reference to a possible embodiment of the invention, but are not intended to limit the scope of the invention.
Fig. 1 shows a schematic diagram of the system according to the invention, with the system in the working position: a) left profile view; (b) top view.
Fig. 2 shows a schematic diagram of the system according to the invention, where: a) the system attached to a stereotactic frame; b) schematic diagram of the system according to the invention.

### DETAILED DESCRIPTION OF THE INVENTION

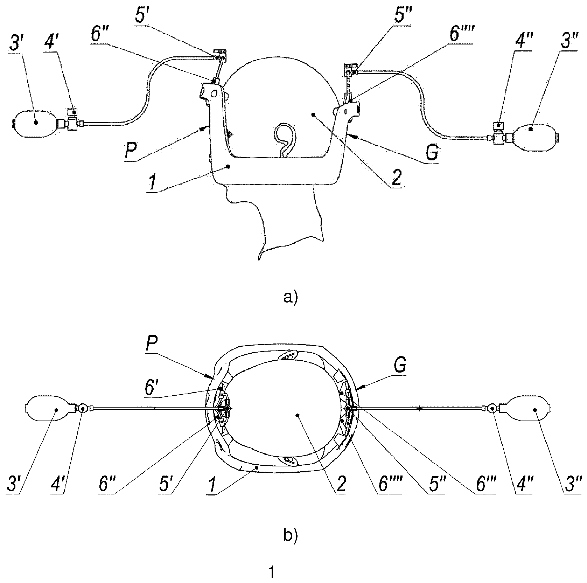

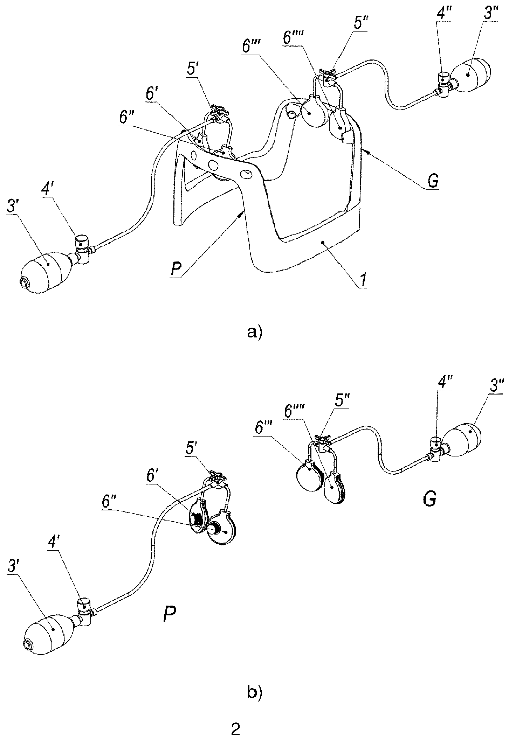

The system for positioning a stereotactic frame (1) on a human head (2) according to an embodiment of the invention and Fig. 1a, 1b , Fig. 2a, 2b, comprises hand pumps (3', 3") comprising air supply-discharge valves (4', 4") and air flow distributors (5', 5"), inflatable soft silicone contact cushions (6', 6", 6"', 6"") which act as pneumatic controls.

With the help of hand pumps (3', 3"), compressed air can be supplied and released through the air flow distributors (5', 5"), to and from inflatable soft silicone contact cushions (6', 6", 6", 6ʺʺ), attached to inside side of front part (P) and to inside side of back part (G) of the stereotactic frame (1).

During positioning of the stereotactic frame (1), the stereotactic frame (1) is fixed on a human head (2) using soft silicone contact cushions (6', 6", 6‴, 6ʺʺ) on the inside side of the frame (1) - two soft silicone contact front cushions (6', 6") on the front side (P) of the stereotactic frame (1) and two soft silicone contact back cushions (6‴, 6"") on the rear side (G) of the stereotactic frame (1). The degree of inflation of the silicone contact cushions (6', 6", 6‴, 6"") is controlled using hand pumps (3', 3"), air supply-discharge valves (4', 4") and air flow distributors (5', 5"). Air is compressed using hand pumps (3', 3"). Air from the cushions (6', 6", 6‴, 6"") may be discharged to alter the hardness of the cushions using the supply-discharge valves (4', 4"). Due to frictional forces between a patient's head and the cushions (6', 6", 6‴, 6ʺʺ) the stereotactic frame (1) can be precisely positioned relative to the position of the patient's head (2) during treatment or surgery procedures.

The stereotactic frame (1) positioning system according to the invention simplifies structure and adjustment of the stereotactic frame (1) on a human head (2) during treatment or surgical procedures, thereby increasing effectiveness of a treatment.

## Claims

1. A stereotactic frame positioning system, comprising means for attaching the stereotactic frame to a human head, **characterized in that** the means for attaching the stereotactic frame (1) to a human head are inflatable and soft silicone contact cushions (6', 6", 6‴, 6""), wherein two said soft silicone contact front cushions (6', 6") are attached to inside side of a front side (P) of the stereotactic frame (1) and two soft silicone contact rear cushions (6‴, 6"") are attached to inside side of the rear side (G) of the stereotactic frame (1), where the pair of the contact cushions (6', 6") on the front side (P) part are connected to one hand pump (3') comprising air supply-discharge valves (4', 4") and air flow distributors (5', 5") for control of inflation of the contact cushions (6', 6"), and the pair of the contact cushions (6‴, 6"") on the rear side (G) is connected to one other hand pump (3") comprising air supply-discharge valves (4', 4") and air flow distributors (5', 5") for control of inflation of the contact cushions (6‴, 6"").

2. The stereotactic frame positioning system according to claim 1, **characterized in that** flow of compressed air in the contact cushions (6', 6", 6‴, 6"") is controlled by air flow distributors (5', 5"), for each cushion independently.
